# EUROPÄISCHE PATENTSCHRIFT

(11) **EP 3 753 260 B1**
(45) Veröffentlichungstag und Bekanntmachung des Hinweises auf die Patenterteilung: **09.07.2025**
(21) Anmeldenummer: 19708950.1
(22) Anmeldetag: 15.02.2019
(51) Int. Cl.: H04R 1/10, H04R 25/00, A61F 11/00, G02C 11/00, A61H 7/00

(54) **VORRICHTUNG ZUR BEHANDLUNG EINES TINNITUS-LEIDENS**
DEVICE FOR TREATING A TINNITUS AFFLICTION
DISPOSITIF DE TRAITEMENT D'UN ACOUPHÈNE

(30) Priorität: 15.02.2018 AT 501442018
(43) Veröffentlichungstag der Anmeldung: 23.12.2020
(73) Patentinhaber: Grübl, Klaus, 5280 Braunau/Inn (AT)
(72) Erfinder: Grübl, Klaus, 5280 Braunau/Inn (AT)
(74) Vertreter: Gassner, Birgitta
(86) Internationale Anmeldenummer: PCT/EP2019/053766
(87) Internationale Veröffentlichungsnummer: WO 2019/158674

(56) Entgegenhaltungen:
- AT-U1- 8 023
- AT-U1- 8 023
- JP-A- H0 956 774
- JP-A- H03 142 414
- JP-U- 3 164 048
- JP-U- 3 164 048

## Beschreibung

### Technisches Gebiet

Die Erfindung betrifft eine Vorrichtung zur Behandlung eines Tinnitus-Leidens.

### Stand der Technik

Der Tinnitus ist eine auditive Wahrnehmung, die zusätzlich zu dem auf das Ohr einwirkenden Schall ein- oder beidseitig wahrgenommen wird. Diese Wahrnehmung beruht auf einer Störung der Hörfunktion. Die Art der scheinbaren Geräusche ist sehr vielfältig: Die auditiven Eindrücke werden als Brummton oder Pfeifton, Zischen, Rauschen, Knacken oder Klopfen beschrieben.

Tinnitus beeinträchtigt die Lebensqualität. Bisherige Behandlungsmethoden (dazu gehören verschiedene Formen der akustischen Stimulation, verhaltenstherapeutische Ansätze, kombinierte Therapieansätze, die akustische Stimulation und verhaltenstherapeutische Elemente beinhalten (zum Beispiel die Tinnitus-Retraining-Therapie), medikamentöse Therapieverfahren, Physiotherapie, magnetische und elektrische Gehirnstimulationsverfahren) greifen nicht immer, oder nicht gut genug. Für die meisten der angebotenen Therapien liegt kein Wirknachweis durch ausreichend große Placebo kontrollierte Studien vor.

Im Stand der Technik ist beispielsweise eine Ohrvorrichtung beschrieben, die schnelle Druckänderungen aufgrund von schnellen Höhenänderungen in Flugzeugen ausgleichen soll um Ohrschmerzen zu vermeiden. GB2450931 beschreibt beispielsweise Ohrenschützer, in denen durch Verwendung einer Luftpumpe der Druck reguliert werden kann.

Bei Gehörschützern, die als Schallschutz getragen werden, tritt nach längerem Gebrauch ein Wärmestau auf, der zu einer übermäßigen Transpiration führen kann. Um einen Luftaustausch zu ermöglichen, ist eine Belüftungsöffnung auf der Kapsel des Gehörschutzes vorgesehen. Durch Veränderung des Volumens des Kapselinnenraums wird so der Luftaustausch ermöglicht (DE2910315).

In CN205598091 (U) wird eine Vorrichtung, umfassend eine Brille, ein Bluetooth-Headset und Kopfhörer, beschrieben. Über diese Vorrichtung kann beispielsweise mittels einer Software Umgebungsklänge, Töne oder Musik zur Behandlung von Tinnitus eingespielt werden.

WO2015164889 beschreibt einen Kopfhörer der im Gebrauch innerhalb oder nahe eines Ohrkanals eines Benutzers angeordnet werden kann und eine Ohrschleife um den Ohrhörer am Ohr des Benutzers zu befestigen. Die Ohrschleife umfasst ein eine aufblasbare Blase um den Ohrhörer im Gebrauch besser am Ohr des Benutzers zu befestigen. US20170303031 beschreibt Kopfhörer umfassend ein aufblasbares Montagesystem um das Gehäuse des Kopfhörers besser am menschlichen Ohr zu befestigen. CN206743497 beschreibt einen Kopfhörer mit einem aufblasbaren Schwellkörper, der jedoch im Innenohr angebracht wird.

JP H09 56774 A offenbart eine Vorrichtung zur Behandlung eines Tinnitus-Leidens umfassend einen Bügel, der Druck auf die Akupunkturpunkte vor dem Ohr ausübt. JP H03 142414 beschreibt eine Brille, die magnetische Körper aufweist um beispielsweise die Blutzirkulation zu verbessern. JP3164048U beschreibt ein Hörgerät, welches die Stellung der Ohrmuschel verändert, um so das Hörempfinden zu verbessern. AT 8 023 U1 beschreibt eine Ohrenklammer zur Lagestabilisierung einer Brille am Kopf und zur Korrektur abstehender Ohren, wobei mittels der Ohrenklammer die Brille am Kopf des Trägers befestigt wird. und der abstehende Ohrrand in Richtung Kopf gedrückt wird

Tinnitus kann durchaus heilbar sein. Insbesondere bei akutem Tinnitus sind die Aussichten auf Heilung gut. Allerdings gibt es keine genauen Zahlen darüber, wie viele Tinnitusbetroffene in welcher Form geheilt werden. Als geheilt kann sich ein Tinnituspatient betrachten, wenn sein Ohrgeräusch verschwunden ist. Es besteht daher nach wie vor der Bedarf nach einer zielgerichteten, dauerhaften und erfolgreichen Behandlung von Tinnitus. Aufgabe der vorliegenden Erfindung ist es daher, eine Vorrichtung und deren Verwendung bereitzustellen, welche nicht die Nachteile und Mängel des Stands der Technik aufweist. Insbesondere ist es Aufgabe der vorliegenden Erfindung ein Verfahren zur Behandlung eines Tinnitus-Leidens zur Verfügung zu stellen.

### Kurze Zusammenfassung der Erfindung

Die Aufgabe wird durch die unabhängigen Ansprüche gelöst. Die vorliegende Erfindung umfasst somit eine neue Vorrichtung zur Behandlung von Tinnitus, wobei diese Vorrichtung die Stellung der Ohrmuschel verändert und so der Schalleintrittswinkel geändert wird. Durch diese Änderung am Außenohr wird das Tinnitus-Leiden gemildert und/oder ganz geheilt.

Die Erfindung ist im beigefügten Anspruchssatz beschrieben.

Insbesondere betrifft die vorliegende Erfindung eine Vorrichtung zur Behandlung eines Tinnitus-Leidens umfassend einen Druckpunktkörper, wobei die Vorrichtung so bemessen ist, dass sie an der Ohrmuschel (Außenohr) befestigt werden kann, dadurch gekennzeichnet, dass der Druckpunktkörper mindestens ein Befestigungselement und ein Druckelement aufweist, wodurch die Stellung der Ohrmuschel (Außenohr) verändert wird.

Eine Ausführungsform umfasst die Vorrichtung wie hierin beschrieben, wobei der Druckpunktkörper eine fixe Größe aufweist oder variabel befüllbar ist.

Eine Ausführungsform umfasst die Vorrichtung wie hierin beschrieben, wobei der Druckpunktkörper ein Gelkissen ist.

Eine Ausführungsform umfasst die Vorrichtung wie hierin beschrieben, wobei der variabel befüllte Druckpunktkörper ein Luftpolsterkissen ist.

Eine Ausführungsform umfasst die Vorrichtung wie hierin beschrieben, wobei das Lustpolsterkissen individuell befüllbar ist.

Eine Ausführungsform umfasst die Vorrichtung wie hierin beschrieben, wobei die Vorrichtung weiters eine Einrichtung zum individuellen Anpassen des Druckpunktkörpers umfasst.

Eine Ausführungsform umfasst die Vorrichtung wie hierin beschrieben, wobei diese Einrichtung eine Luftpumpe, ein Kompressor oder eine Spritze ist.

Eine Ausführungsform umfasst die Vorrichtung wie hierin beschrieben, wobei die Vorrichtung weiters eine Steuereinheit mit Speicher umfasst.

Eine Ausführungsform umfasst die Vorrichtung zur Behandlung eines Tinnitus-Leidens, bestehend aus der Vorrichtung, die so bemessen ist, dass sie an der Ohrmuschel befestigt werden kann, und mindestens einen Druckpunktkörper aufweist, und optional eine Einrichtung zum individuellen Anpassen des variablen Druckpunktkörper an seinen Träger, sowie eine elektronische Steuereinheit mit Speicher.

Weiter wird die Verwendung der erfindungsgemäßen Vorrichtung zur Behandlung eines Tinnitus-Leidens beschrieben.

Die erfindungsgemäße Vorrichtung ist zur Behandlung eines Tinnitus-Leidens geeignet, wobei durch die Vorrichtung, insbesondere durch den Druckpunktkörper, die Stellung der Ohrmuschel (Außenohr) verändert wird.

Die erfindungsgemäße Vorrichtung ist zur Behandlung eines Tinnitus-Leidens geeignet, da durch die Vorrichtung, insbesondere durch das Druckelement, der Eintrittwinkels des Schalls am Außenohr verändert wird.

Die erfindungsgemäße Vorrichtung ist zur Behandlung eines Tinnitus-Leidens geeignet, da durch die Vorrichtung, insbesondere durch den Druckpunktkörper, Druck auf die Ohrmuschel (Außenohr) ausgeübt wird. Durch diesen Druck kann das Mittelohr deformiert und der Schall anders gebrochen werden.

Weiter wird ein Verfahren zur Behandlung eines Tinnitus-Leidens beschrieben, umfassend eine Vorrichtung, die hinter der Ohrmuschel platziert ist, wobei diese Vorrichtung derart ausgestaltet ist, dass sie die Stellung der Ohrmuschel des Außenohrs verändert wird.

Weiters wird ein Verfahren zur Behandlung eines Tinnitus-Leidens hierin, wobei durch diese Vorrichtung, insbesondere durch den Druckpunktkörper, Druck auf die Ohrmuschel und/oder auf das Mittelohr ausgeübt wird.

Weiters wird ein Verfahren zur Behandlung eines Tinnitus-Leidens hierin, wobei jeweils hinter beiden Ohrmuscheln eine Vorrichtung, insbesondere ein Druckpunktkörper platziert ist, wobei insbesondere durch den Druckpunktkörper, Druck auf die Ohrmuscheln ausgeübt wird.

Weiters wird ein Verfahren zur Behandlung eines Tinnitus-Leidens beschrieben, wobei mindestens eine Vorrichtung wie hierin beschrieben hinter einer oder beiden Ohrmuscheln platziert ist.

### Kurze Beschreibung der Zeichnungen

Abbildung 1 zeigt eine schematische Darstellung des Ohrs.
Abbildung 2 zeigt eine schematische Darstellung des Ohrs mit Schalleintritt.
Abbildung 3 zeigt eine schematische Darstellung des Ohrs mit verändertem Schalleintritt.
Abbildung 4 zeigt eine schematische Grafik einer Brille.
Abbildung 5 zeigt eine schematische Grafik eines Brillenbogens mit Luftkammern und Steuerungshardware.
Abbildung 6 zeigt einen schematischen Druckpunktköper.
Abbildung 7 zeigt eine schematische Darstellung eines Bügels mit einem Druckpunktkörper der individuell auf dem Bügel fixiert werden kann.
Abbildung 8 zeigt eine schematische Darstellung eines Bügels mit Luftkammern und Steuerungshardware.
Abbildung 9A und 9B zeigen den Trend der subjektiven Wahrnehmung der Studienteilnehmer während des Studienzeitraums. Die Studienteilnehmer mussten ihre individuelle Beurteilung ihres Tinnitus-Leidens über den Studienzeitraum bewerten, wobei ihr subjektives Empfinden gemäß einer Werteskala von 0 = für nicht belastend/nicht hörbar bis 10 = stark belastend/sehr laut eingetragen wurde. Die Trendlinien wurde logarithmisch in Excel anhand der Einzeldaten pro TN errechnet.
Abbildung 10 zeigt einen Fragebogen.

### Detaillierte Beschreibung der Erfindung

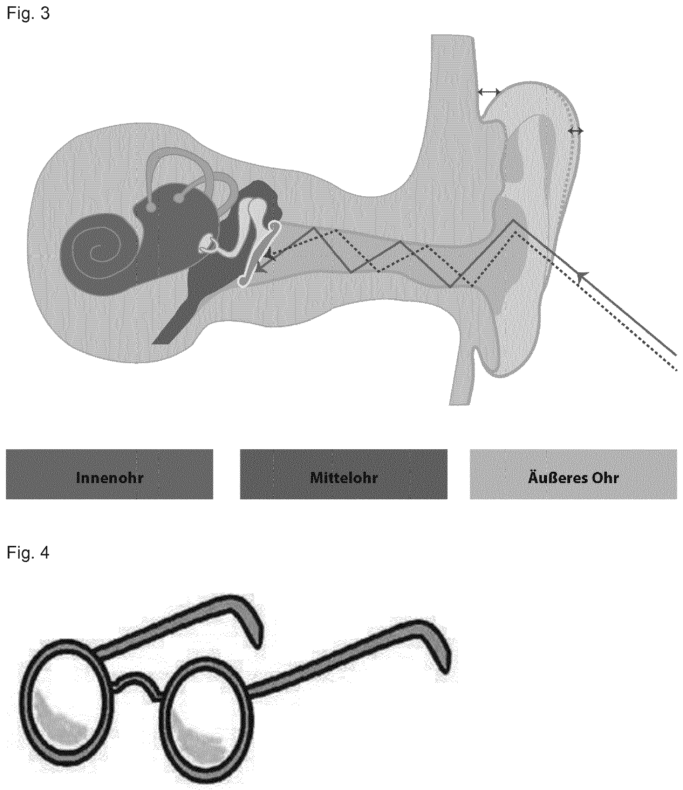

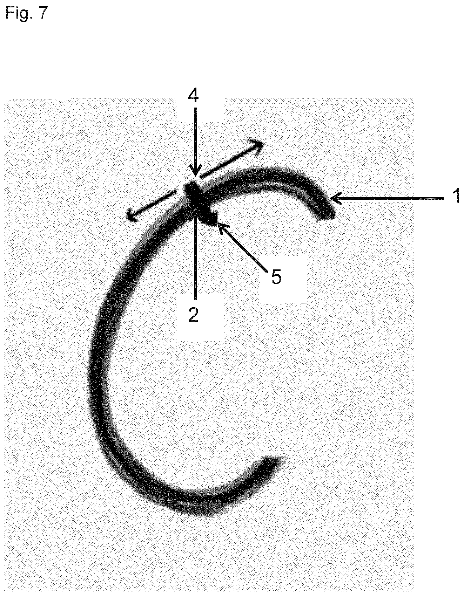

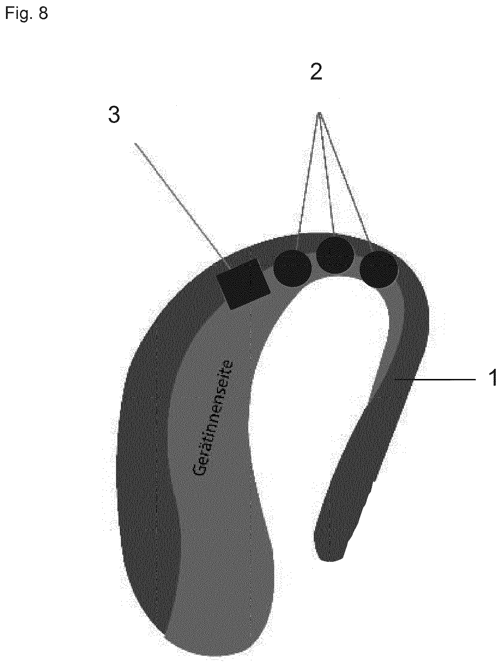

Das erfindungsgemäße Ausführungsbeispiel wird in Abbildung 7 gezeigt. Die in Abbildung 4, 5, und 8 gezeigten Ausführungsbeispiele fallen nicht unter den Anspruchsgegenstand.

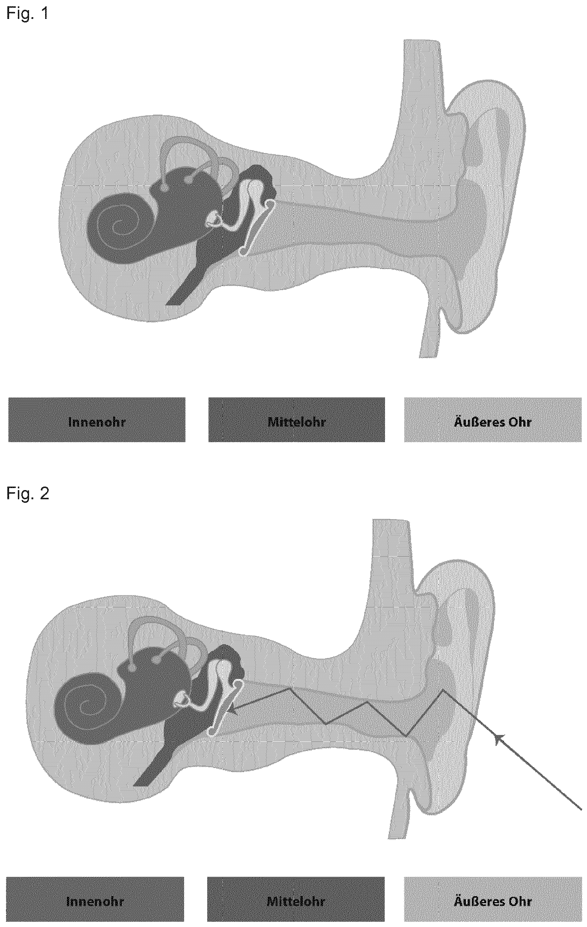

Das Ohr besteht aus dem Außen-, Mittel- und Innenohr (siehe Figur 1).

Das Außenohr umfasst den Ohrknorpel, die Ohrmuschel, das Ohrläppchen und den äußeren Gehörgang oder auch Ohrkanal und die Außenseite des Trommelfells. Es dient nicht nur dem Einfangen des Schalls, sondern auch dazu, eine bestimmte Einfallsrichtung des Schalls durch spektrale Minima und Maxima zu kodieren (siehe Lokalisation). Die zahlreichen Erhebungen und Vertiefungen der Ohrmuschel bilden akustische Resonatoren, die jeweils bei Schalleinfall aus einer bestimmten Richtung angeregt werden. Hierdurch entstehen richtungsabhängige Minima und Maxima im Frequenzspektrum des Ohrsignals, die vom Gehör zur Bestimmung der Einfallsrichtungen oben, unten, vorn oder hinten genutzt werden (Richtungsbestimmende Bänder).

Zum Mittelohr gehören das Trommelfell und die Gehörknöchelchen Hammer, Amboss und Steigbügel. Das Runde Fenster verbindet die Paukentreppe des Innenohrs mit dem Mittelohr. Die Eustachische Röhre, auch Ohrtrompete genannt, verbindet Mittelohr und Nasenrachenraum. Im Mittelohr findet eine mechanische Impedanzwandlung statt, die eine optimale Übertragung des Signals vom Außenohr zum Innenohr ermöglicht. Da die akustische Impedanz von Wasser ca. 3000-mal so groß ist wie die von Luft, würde ohne das von den Gehörknöchelchen gebildete Hebelsystem nur ein geringer Teil der Schallenergie, die das Trommelfell erreicht, an das Innenohr weitergegeben werden.

Das Innenohr liegt in einem kleinen Hohlraumsystem (knöchernes Labyrinth) innerhalb des Felsenbeines, eines Teils des Schläfenbeines. In diesem knöchernen Labyrinth befindet sich das membranöse oder häutige Labyrinth, bestehend aus der Gehörschnecke, in der Schall in Nervenimpulse umgesetzt wird, und dem Gleichgewichtsorgan. Das Gleichgewichtsorgan besteht aus den Bogengängen und zwei bläschenförmigen Anteilen, dem Utriculus und dem Sacculus. Es dient dem Erkennen von Bewegungsänderungen und der Richtung der Erdanziehungskraft. Gehörschnecke und Gleichgewichtsorgan sind ähnlich gebaut: Beide sind mit zwei gemeinsamen parallelen Flüssigkeitssystemen (Perilymphe und Endolymphe) gefüllt und besitzen Haarzellen. Die Haarzellen sind zylinderförmig und haben ihren Namen von den etwa 30 bis 150 haarartigen Fortsätzen am oberen Ende der Zelle (Stereozilien). Durch Bewegungen der Flüssigkeit werden die Härchen gebogen und lösen dabei Nervenimpulse aus. Am unteren Ende befindet sich eine Synapse mit einem sensorischen Neuron. Diese schüttet schon im Ruhezustand Neurotransmitter aus. Werden nun durch Schallschwingungen oder Bewegungsänderungen des Kopfes die Haarfortsätze ausgelenkt, ändert sich die Menge der Neurotransmitter. Im Gleichgewichtsorgan sind die Haarfortsätze mit einer Art Gallertschicht überzogen, auf die kleine Kristalle von Calciumcarbonat aufgelagert sind, welche die Auswirkung von Bewegungen verstärken. Von der Gehörschnecke geht der Hörnerv gemeinsam mit den Nervenbündeln des Gleichgewichtsorganes als Nervus vestibulocochlearis in Richtung Gehirn.

Die Auswirkungen eines Tinnitus richten sich stark nach der subjektiven Wahrnehmung und Beurteilung der Ohrgeräusche. Diese können einseitig oder beidseitig auftreten.

Tinnitus aurium bedeutet "das Klingeln der Ohren". Medizinisch definiert ist der Tinnitus als akustische Wahrnehmung, die ohne entsprechenden akustischen Reiz von außerhalb des Körpers entsteht und keinen Informationsgehalt besitzt.

Prinzipiell werden zwei Formen unterschieden. Beim objektiven Tinnitus gibt es eine körpereigene Schallquelle im Ohr oder in der Nähe des Ohrs, deren Schallaussendungen wahrgenommen werden. Das heißt, die oft von den Blutgefäßen oder der Muskulatur ausgehenden Geräusche existieren tatsächlich und sind somit auch für andere hörbar, wenn auch meist nur mit dem Stethoskop oder anderen medizinischen Geräten.

Sehr viel häufiger ist aber der subjektive Tinnitus. Hier nehmen die Betroffenen Töne und Geräusche wahr, die sich nicht auf eine physikalische Schallquelle zurückführen lassen und deshalb auch für andere Menschen nicht zu hören sind. Das bedeutet aber keineswegs, dass die Patienten sich das Brummen, Summen, Pfeifen, Klingeln, Rauschen oder Klopfen nur einbilden. Der subjektive Tinnitus ist vielmehr auf eine fehlerhafte Informationsbildung bzw. -verarbeitung im auditorischen System zurückzuführen, das sich vom Ohr über den Hörnerv bis zu den Hörzentren im Gehirn erstreckt.

Bei vielen Betroffenen lässt sich allerdings gar nicht definitiv feststellen, worauf die Ohrgeräusche zurückzuführen sind. Dies wird als idiopathischer Tinnitus bezeichnet.

Überraschenderweise wurde nun gefunden, dass durch Veränderung der Stellung des Außenohrs zum restlichen Ohr der Schall verändert wird, das heißt, der Schall wird im Vergleich zum "Normaleintritt" gebrochen. Dieses führt zu einem anderen Auftrittspunkt am Trommelfell. Dadurch wird der Hammer(griff) des ersten Gehörknochens anders bewegt, und sendet veränderte Drucksignale an die nächsten Gehörknochen bzw. dann weiter an die Gehörschnecke. In der Gehörschnecke werden die in einer Flüssigkeit gelagerten Sinneshärchen verändert in Bewegung versetzt. Dieses führt zu einer veränderten Umwandlung der elektrischen Signale ins Gehirn und zu einer lerntechnische Veränderung der Synapsen - die bisherigen Töne werden nicht mehr gehört und somit langfristig "verlernt".

Durch Veränderungen des Schalleintrittswinkels werden die als "störend" wahrgenommenen Ohrgeräusche (Tinnitus) nicht mehr wahrgenommen, da der Schall auf anderen Auftrittspunkten am Trommelfell auftrifft.

Schall wird durch andere Brechung (Weglängenänderung) anders frequent. Frequenzänderung durch Abstandsänderung zwischen Beobachter (Trommelfell) und Schallquelle (Doppler Effekt). Der Schallweg im Ohr zwischen Außenohr und Trommelfell verändert sich. Daher werden die als störend empfundenen bisherigen "alten" Frequenzen nicht mehr wahrgenommen, da sich diese "frequent" verändert haben, und im Gehirn nicht mehr wahrgenommen werden. Dieses wird sich hauptsächlich bei hohen Frequenzen ergeben.

Mit der erfindungsgemäßen Vorrichtung wird der Schalleintrittswinkel so verändert, dass die Betroffenen die bisher gelernten störenden Töne nicht mehr hören. Die erfindungsgemäße Vorrichtung umfasst daher einen Druckpunktkörper, der so bemessen ist, dass er an der Ohrmuschel befestigt werden kann, und mindestens ein Druckelement aufweist, wobei durch den Druckpunktkörper die Stellung der Ohrmuschel verändert wird. Durch die Änderung der Stellung des Außenohrs durch diesen Druckpunktkörper wird daher der Eintrittswinkel des Schalls in das Ohr verändert.

Insbesondere ist es vorteilhaft durch den Druckpunktkörper Druck auf das Außenohr auszuüben. Dieser Druck kann dazu führen, dass die Stellung des Außenohrs derart verändert wird, dass der Eintrittswinkel des Schalls ebenfalls geändert wird und so störende Tinnitus-Töne nicht mehr gehört werden. Die Wahrnehmung von Geräuschen die Ihre Ursache nicht in akustischen Signalen aus der Umwelt haben wird mit der erfindungsgemäßen Vorrichtung gemindert beziehungsweise gänzlich ausgeschaltet.

Der punktuelle Druckpunktkörper kann auf jede herkömmliche Vorrichtung angebracht werden, die an der Ohrmuschel befestigt werden kann. Abhängig von der Ausführungsform der Vorrichtung können ein oder mehrere punktuelle Druckpunktkörper angebracht werden.

Herkömmliche Vorrichtung sind beispielsweise Brillengestelle oder einfache Ohrbügel, wie sie beispielsweise bei Kopfhörern oder Hörgeräte eingesetzt werden. Die erfindungsgemäße Ausführungsform ist ein einfacher Bügel oder eine bügelähnliche Halterung, der am Außenohr befestigt werden kann.

Der Begriff "punktuell" wie hierin verwendet bezieht sich auf eine einzelne Stelle der Vorrichtung, an der ein Druckpunktkörper angebracht wird. Insbesondere Ohrhörer können bereits einen Schwellkörper aufweisen, der beispielsweise ringförmig ausgebildet ist und so das gesamte Ohr abdeckt. Punktuell dazu können weiters ein oder mehrere Druckpunktkörper angebracht werden, sodass sich die Stellung der Ohrmuschel individuell verändern lässt.

Der Druckpunktkörper kann aus unterschiedlichen Materialien hergestellt werden. Prinzipiell kann jedes Material verwendet werden, welches geeignet ist, die übliche Stellung der Ohrmuschel zu verändern. Beispielsweise kann der Druckpunktkörper aus Kunststoff, Stoff, Filz, Schaumstoff, Gel, Gummi, elastischen Klebestreifen oder ähnlichen Materialien gefertigt sein. Der Druckpunktkörper kann aus einem aufgeschäumten Material; aus einem elastischen, deformierbaren Material, oder aus einem Plastikmaterial, beispielsweise aus Polypropylen hergestellt werden.

Der Druckpunktkörper kann eine fixe oder ein variable Form aufweisen. In einer Ausführungsform besteht der Druckpunktkörper aus einem Befestigungselement und einem Druckelement. Insbesondere kann der Druckpunktkörper auch aus einem Druckelement bestehen, wobei das Befestigungselement als Haftseite des Druckelements ausgebildet ist. In dieser Ausführungsform wird der Druckpunktkörper direkt hinter der Ohrmuschel mittels der Haftseite befestigt. Die Haftseite kann zu diesem Zweck eine Oberflächenbeschichtung aufweisen, wie beispielsweise eine Gummierung oder eine Haft- oder Klebebeschichtung.

Vorzugsweise ist das Befestigungselement zylinderförmig mit einer kreisförmigen Querschnittsgeometrie. Das Befestigungselement kann im Querschnitt allerdings auch oval, dreieckig, rechteckig, quadratisch, dreieckig, mehreckig, L-förmig oder dergleichen sein. Das heißt, das Befestigungselement kann im Querschnitt jede beliebige Geometrie aufweisen. Hierdurch kann das Befestigungselement an beliebigen Stellen auf der Vorrichtung angebracht werden und so den individuellen Bedürfnissen des Trägers angepasst werden. In einer weiteren Ausführungsform kann das Befestigungselement mittels eines Klipp-Mechanismus an der Vorrichtung fixiert werden.

Eine weitere Ausführungsform des Druckpunktkörpers umfasst auch ein Druckelement, welches ein fixes oder ein variables Volumen aufweisen kann. Für Druckelemente mit einem variablen Volumen sind beispielsweise Polster, die je nach Bedarf mit Luft, Wasser, und/oder generell mit Flüssigkeiten befüllt werden, und so optimal an den Bedarf des Trägers angepasst werden können, besonders geeignet.

Druckelemente mit einem fixen Volumen und einer fixen Form sind vorzugsweise aus Kunststoff gefertigt.

Eine weitere Ausführungsform umfasst daher die erfindungsgemäße Vorrichtung, die neben dem Druckpunktkörper und dem variable anzupassenden Druckelement auch eine Miniaturluftpumpe, eine (Miniatur)Spritze oder einen Miniaturkompressor zum individuellen Befüllen des Druckelements enthält. Der Miniaturkompressor kann durch Hin- und Herbewegen, durch Rotationsschrauben, durch schneckenförmiges Bewegen, durch Zentrifugieren oder anderen Verfahren arbeiten. Eine Ausführungsform umfasst auch eine thermische Veränderung des Druckelements, beispielsweise durch Reiben, Kneten, Drücken (also mechanische Einwirkungen), durch Hinzuführen oder Ableiten von Temperatur oder den Einsatz von Mikropumpen mit einer Piezomembran.

Um die Einstellung des variablen Druckelements optimal anzupassen, kann es vorteilhaft sein, wenn die Vorrichtung eine Steuereinheit aufweist, mit der der Schwellkörper individuell an die Bedürfnisse des Trägers angepasst werden kann.

Die Steuereinheit kann weiters einen Speicher aufweisen, in dem die Statusinformation des Schwellkörpers gespeichert wird.

### Ausführungsbeispiele

Die nachfolgenden Ausführungsbeispiele illustrieren die vorliegende Erfindung ohne sie jedoch in ihrem Umfang zu beschränken

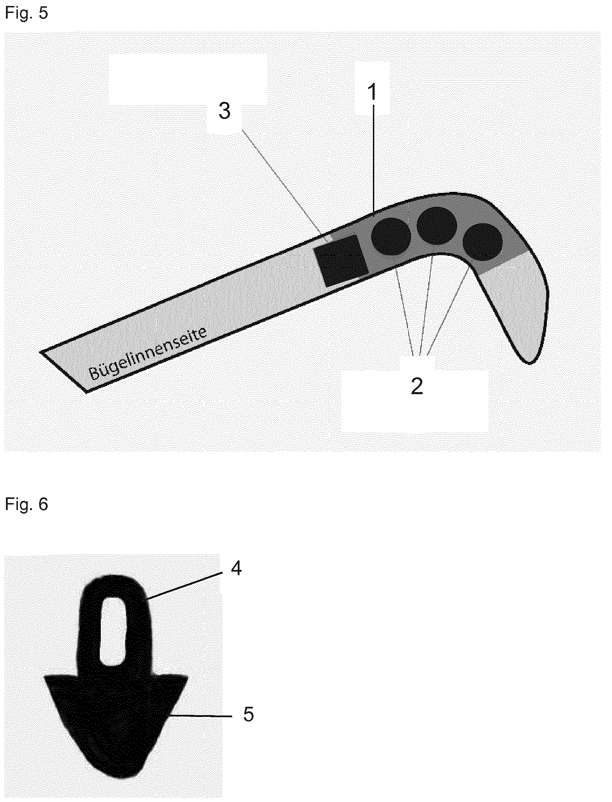

Figur 5 zeigt einem Brillenbügel (1), der auf der Bügelinnenseite drei variable Druckpunktkörper (2) aufweist. Diese variablen Druckpunktkörper können mit dem Kompressor (3) individuell befüllt werden.

Ein Druckpunktkörper (2) ist schematisch in Figur 6 dargestellt. Figur 6 zeigt einen Druckpunktkörper (2) bestehend aus einem Befestigungselement (4) und einem Druckelement (5).

Figur 7 zeigt das erfindungsgemäße Ausführungsbeispiel bestehend aus einem Bügel mit einem Druckpunktkörper. Dieser Druckpunktkörper (2) bestehend aus einem Befestigungselement (4) und Druckelement (5) kann je nach den Bedürfnissen des Trägers individuell angepasst werden, wobei der Druckpunktkörper (2) auf dem Bügel entsprechend fixiert wird um eine optimale und individuelle Druckausübung zu gewährleisten.

### Fallstudien

Unter der Kontrolle des Erfinders und Anmelders wurde bereits eine erste Fallstudie durchgeführt. Die teilnehmenden Personen mussten folgende Voraussetzungen für die Studienteilnahme erfüllen:
- Personen, die bezüglich ihres Tinnitus-Leidens als bereits medizinisch austherapiert gelten. Das heißt, Personen die auf keine mögliche Therapie mehr reagierend;
- Brillenträger; und
- Personen, die sich bereit erklärten 2 mal täglich an einer Onlinebefragung teilzunehmen.

In Summe nahmen 7 Teilnehmer, die in einer Arztpraxis von einem Allgemeinmediziner ausgewählt wurden, an dieser ersten Fallstudie teil. An den Brillenbügel dieser Studienteilnehmer wurde ein Druckpunktkörper fixiert. Diese erfindungsgemäße Vorrichtung mit den Druckpunktkörpern wurde von den Studienteilnehmern zum überwiegenden Teil den ganzen Tag getragen.

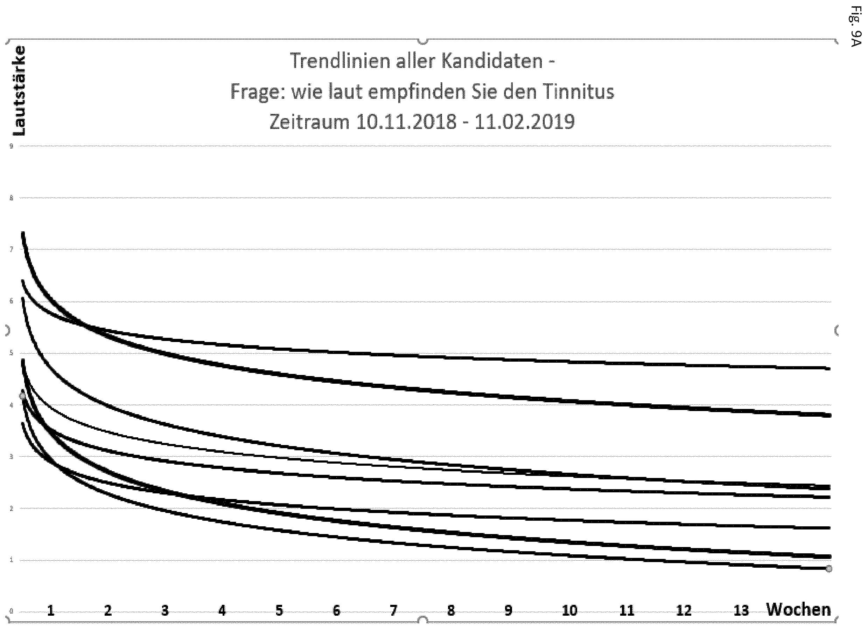

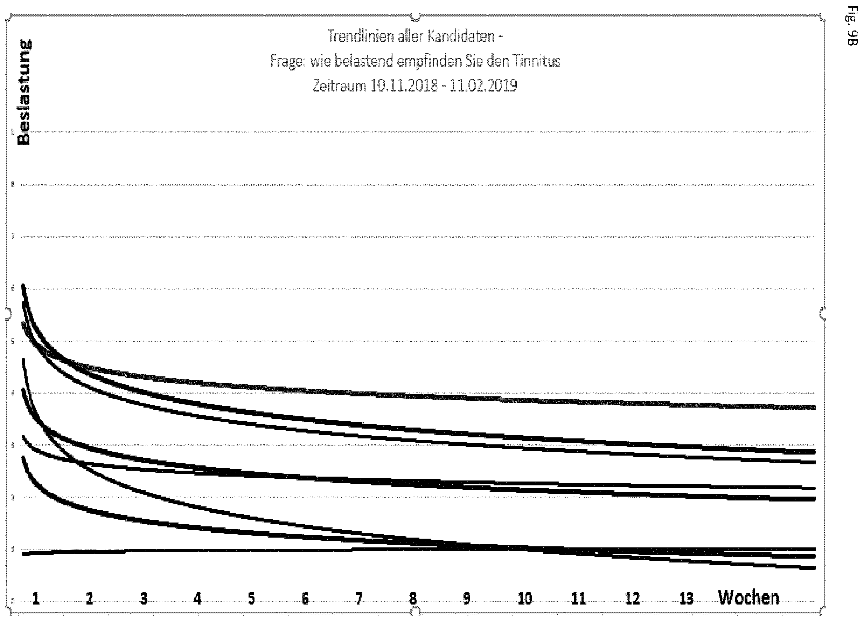

Bei 70% der Patienten hat sich innerhalb des Studienzeitraums von ca. 8 Wochen eine signifikante Verbesserung des subjektiv wahrgenommenen Tinnitus-Leidens ergeben (siehe Figur 9). Dazu wurde die Onlinebefragung der Studienteilnehmer über diesen Zeitraum ausgewertet. Die Studienteilnehmer wurden nach dem persönlichen Empfinden der Lautstärke des Tinnitus gefragt. Dabei sollten sie ihre persönliche Einschätzung in einer Werteskala von 0 = gar nicht zu hören bis 10 = sehr stark zu hören einordnen. Ebenso sollten sie die persönliche Belastung ihres Tinnitus in dieser Werteskala gleichfalls von 0 = gar nicht bis 10 = sehr stark belastend einordnen.

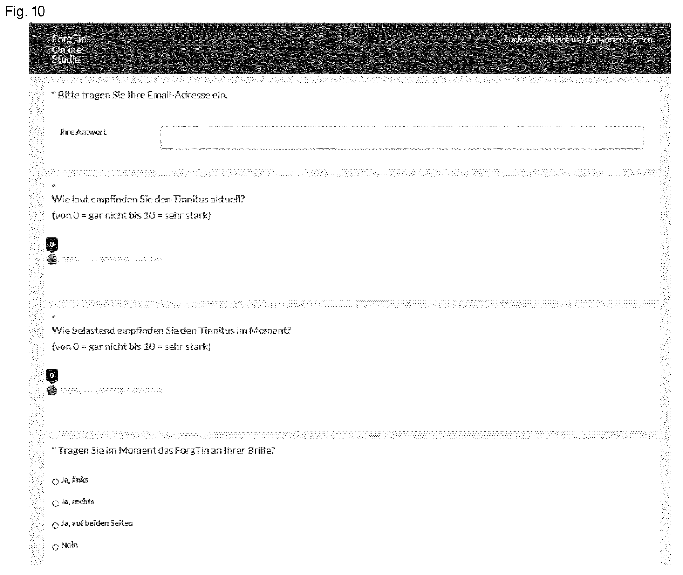

Die Teilnehmer beantworteten folgenden Fragebogen in Figur 10 online, wobei ForgTir den Druckpunktkörper bezeichnet.

## Patentansprüche

1. Vorrichtung zur Behandlung eines Tinnitus-Leidens bestehend aus einem Ohrbügel (1), welcher nicht Teil eines Brillengestells ist, einem Druckpunktkörper (2) umfassend ein Befestigungselement (4) und ein Druckelement (5), wobei der Druckpunktkörper (2) auf dem Ohrbügel (1) so befestigt ist, dass der Druckpunktkörper (2) die Stellung der Ohrmuschel verändert, **dadurch gekennzeichnet, dass** der Druckpunktkörper (2) variabel auf dem Ohrbügel (1) verschoben werden kann.

2. Vorrichtung nach Anspruch 1, **dadurch gekennzeichnet, dass** das Druckelement (5) eine definierte Größe aufweist.

3. Vorrichtung nach einem der Ansprüche 1 bis 2, **dadurch gekennzeichnet, dass** das Befestigungselement (4) ein ringförmiges Halterungselement ist.

4. Vorrichtung nach einem der Ansprüche 1 bis 3, **dadurch gekennzeichnet, dass** durch den Druckpunktkörper (2) individuell unterschiedliche Punkte der Ohrmuschel stimuliert werden und die Stellung der Ohrmuschel individuell verändert wird.

## Claims

1. Device for treating a tinnitus condition, comprising an ear clip (1) that is not part of a spectacle frame, and a pressure point body (2) comprising a fastening element (4) and a pressure element (5),
wherein the pressure point body (2) alters the position of the auricle,
**characterized in that** the pressure point body (2) is adjustably displaceable along the ear clip (1).

2. Device according to claim 1,
**characterized in that** the pressure element (5) has a defined size.

3. Device according to any one of claims 1 to 2,
**characterized in that** the fastening element (4) is an annular retaining element.

4. Device according to any one of claims 1 to 3,
**characterized in that** the pressure point body (2) stimulates individually different points of the auricle and individually alters the position of the auricle.

## Revendications

1. Dispositif de traitement d'une affection de type acouphène, comprenant une branche auriculaire (1) qui ne fait pas partie d'une monture de lunettes, et un corps de point de pression (2) comprenant un élément de fixation (4) et un élément de pression (5),
le corps de point de pression (2) modifiant la position du pavillon de l'oreille,
**caractérisé en ce que** le corps de point de pression (2) est déplaçable de manière variable le long de la branche auriculaire (1).

2. Dispositif selon la revendication 1, **caractérisé en ce que** l'élément de pression (5) présente une dimension définie.

3. Dispositif selon l'une quelconque des revendications 1 à 2, **caractérisé en ce que** l'élément de fixation (4) est un élément de maintien annulaire.

4. Dispositif selon l'une quelconque des revendications 1 à 3, **caractérisé en ce que** le corps de point de pression (2) stimule des points individuellement différents du pavillon de l'oreille et modifie individuellement la position du pavillon de l'oreille.
